# EUROPEAN PATENT APPLICATION

(11) **EP 4 527 886 A1**
(43) Date of publication of application: **26.03.2025**
(21) Application number: 23807712.7
(22) Date of filing: 19.05.2023
(51) Int. Cl.: C08L 29/10, A61L 31/10, C08F 8/00, C08F 116/18, C08K 9/04, C09C 3/10

(54) **CELL-ADHESIVE COMPOSITION AND POLYMER-COATED MICROPARTICLES**

(30) Priority: 20.05.2022 JP 2022082877
(71) Applicant: Maruzen Petrochemical Co., Ltd., Tokyo 104-8502 (JP); The University of Tokyo, Bunkyo-ku, Tokyo 113-8654 (JP)
(72) Inventor: NISHIURA, Takao, Ichihara-shi, Chiba 290-8503 (JP); MIYATA, Kanjiro, Tokyo 113-8654 (JP); FUJIURA, Kento, Tokyo 113-8654 (JP)
(74) Representative: Regimbeau
(86) International application number: PCT/JP2023/018696
(87) International publication number: WO 2023/224111

(57) **Abstract**

[Problem] Provided is a cell-adhesive composition including an oxyethylene chain-containing polymer having excellent biocompatibility and excellent reactivity.

[Solution] A cell-adhesive composition according to the present invention includes an oxyethylene chain-containing polymer represented by the following general formula (1): 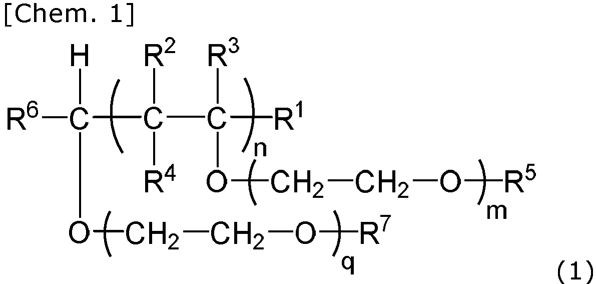 (wherein, in the general formula (1), R¹ is -(CH₂)ₚ-SH, -(CH₂)ₚ-N₃, -(CH₂)ₚ-NH₂, or -(CH₂)ₚ-COOH, and p is an integer of 1 to 5; R² to R⁴ are each independently hydrogen or a C₁₋₁₀ alkyl group; R⁵ is a C₁₋₁₀ alkyl group; R⁶ is hydrogen or a C₁₋₅ alkyl group; R⁷ is a C₁₋₁₀ alkyl group; n is an integer of 5 to 1000; m is an integer of 1 to 10; and q is an integer of 0 to 5).

## Description

### BACKGROUND OF THE INVENTION

### Technical Field

The present invention relates to a cell-adhesive composition containing an oxyethylene chain-containing polymer. In addition, the present invention relates to microparticles coated with an oxyethylene chain-containing polymer.

### Background Art

A vinyl ether polymer is conventionally used as a compounding component of a chemical product such as an adhesive, coating material, or lubricant. It is known that a vinyl ether generally contains an electron-donating substituent, and thus, produces a vinyl ether polymer through cationic polymerization. For the purpose of modifying a vinyl ether polymer, a polymerization method of introducing a functional group into an end has been studied. For example, it is disclosed that an acetal group is introduced into an end of a vinyl ether polymer (see Patent Literature 1 and 2).

In addition, an oxyethylene chain-containing polymer is a material having extremely excellent biocompatibility. To utilize an oxyethylene chain-containing polymer as a biomaterial, it is vital to introduce a highly reactive functional group into the polymer. However, there is no known oxyethylene chain-containing polymer having a highly reactive functional group introduced into an end thereof.

In recent years, polyethylene glycol (PEG) has been utilized most widely as a technique for enhancing the retentivity of a drug delivery system (DDS) in the blood. This is because PEG achieves the effect (stealthiness) of inhibiting the adsorption of protein and the recognition of immunization. It is known, however, that owing to the high stealthiness, PEG simultaneously inhibits the interaction with a target cell (for example, a cancer cell), and thus, significantly decreases the amount of delivery of a drug into a cell. This is called the PEG dilemma, and is one of the important problems in the DDS.

### Citation List

### Patent Literature

Patent Literature 1: JP2003-201315A
Patent Literature 2: JP2004-244535A

### SUMMARY OF INVENTION

### Technical Problem

The present inventors have attempted to develop a DDS, using, instead of PEG, poly(2-methoxyethylvinyl ether) (PMOVE) that is a vinyl ether polymer containing an oxyethylene chain. PMOVE has excellent biocompatibility in the same manner as PEG, and is expected to achieve high stealthiness, but has not been sufficiently utilized as a DDS because a PMOVE having a highly reactive substituent has not been developed.

Accordingly, an object of the present invention is to provide a cell-adhesive composition that contains an oxyethylene chain-containing polymer, and has excellent biocompatibility and excellent reactivity. In addition, an object of the present invention is to provide microparticles that have excellent biocompatibility and are coated with an oxyethylene chain-containing polymer.

### Solution to Problem

The present inventors have vigorously made studies to solve the above-described problems, and consequently completed the present invention through the discovery that using an oxyethylene chain-containing polymer having a highly reactive functional group introduced into one end (ω end) thereof enables the above-described problems to be solved.

That is, the present invention provides the following inventions.
[1] A cell-adhesive composition including an oxyethylene chain-containing polymer represented by the following general formula (1): 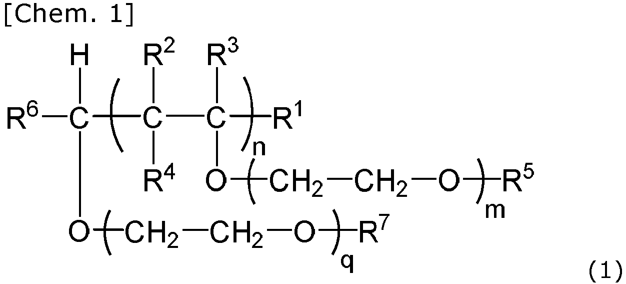
   (wherein, in the general formula (1), R¹ is -(CH₂)ₚ-SH, -(CH₂)ₚ-N₃, -(CH₂)ₚ-NH₂, or -(CH₂)ₚ-COOH, and p is an integer of 1 to 5;
   R² to R⁴ are each independently hydrogen or a C₁₋₁₀ alkyl group;
   R⁵ is a C₁₋₁₀ alkyl group;
   R⁶ is hydrogen or a C₁₋₅ alkyl group;
   R⁷ is a C₁₋₁₀ alkyl group;
   n is an integer of 5 to 1000;
   m is an integer of 1 to 10; and
   q is an integer of 0 to 5).
[2] The cell-adhesive composition according to [1], wherein, in the general formula (1), R¹ is -(CH₂)ₚ-SH, and p is an integer of 1 to 3;
   R² to R⁴ are each independently hydrogen or a C₁₋₄ alkyl group;
   R⁵ is a C₁₋₄ alkyl group;
   R⁶ is a C₁₋₂ alkyl group;
   R⁷ is a C₁₋₇ alkyl group;
   n is an integer of 10 to 500;
   m is an integer of 1 to 5; and
   q is an integer of 0 to 2.
[3] The cell-adhesive composition according to [1], wherein, in the general formula (1), R¹ is -(CH₂)ₚ-N₃, and p is an integer of 1 to 3;
   R² to R⁴ are each independently hydrogen or a C₁₋₄ alkyl group;
   R⁵ is a C₁₋₄ alkyl group;
   R⁶ is a C₁₋₂ alkyl group;
   R⁷ is a C₁₋₇ alkyl group;
   n is an integer of 10 to 500;
   m is an integer of 1 to 5; and
   q is an integer of 0 to 2.
[4] The cell-adhesive composition according to [1], wherein, in the general formula (1), R¹ is -(CH₂)ₚ-NH₂, and p is an integer of 1 to 3;
   R² to R⁴ are each independently hydrogen or a C₁₋₄ alkyl group;
   R⁵ is a C₁₋₄ alkyl group;
   R⁶ is a C₁₋₂ alkyl group;
   R⁷ is a C₁₋₇ alkyl group;
   n is an integer of 10 to 500;
   m is an integer of 1 to 5; and
   q is an integer of 0 to 2.
[5] The cell-adhesive composition according to [1], wherein, in the general formula (1), R¹ is -(CH₂)ₚ-COOH, and p is an integer of 1 to 3;
   R² to R⁴ are each independently hydrogen or a C₁₋₄ alkyl group;
   R⁵ is a C₁₋₄ alkyl group;
   R⁶ is a C₁₋₂ alkyl group;
   R⁷ is a C₁₋₇ alkyl group;
   n is an integer of 10 to 500;
   m is an integer of 1 to 5; and
   q is an integer of 0 to 2.
[6] The cell-adhesive composition according to any one of [1] to [5], wherein the number-average molecular weight (Mn) of the oxyethylene chain-containing polymer is 500 to 100,000.
[7] The cell-adhesive composition according to any one of [1] to [6], wherein the ratio of the weight-average molecular weight (Mw) of the oxyethylene chain-containing polymer to the number-average molecular weight (Mn) of the polymer is 1.0 or more and 3.0 or less.
[8] Microparticles coated with an oxyethylene chain-containing polymer,
   wherein the oxyethylene chain-containing polymer is represented by the following general formula (1): 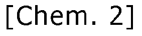 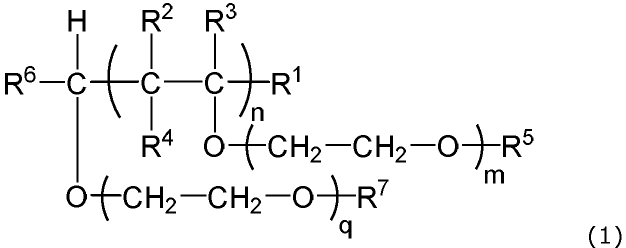
   (wherein, in the general formula (1), R¹ is -(CH₂)ₚ-SH, -(CH₂)ₚ-N₃, -(CH₂)ₚ-NH₂, or -(CH₂)ₚ-COOH, and p is an integer of 1 to 5;
   R² to R⁴ are each independently hydrogen or a C₁₋₁₀ alkyl group;
   R⁵ is a C₁₋₁₀ alkyl group;
   R⁶ is hydrogen or a C₁₋₅ alkyl group;
   R⁷ is a C₁₋₁₀ alkyl group;
   n is an integer of 5 to 1000;
   m is an integer of 1 to 10; and
   q is an integer of 0 to 5).
[9] The polymer-coated microparticles according to [8], wherein, in the general formula (1), R¹ is -(CH₂)ₚ-SH, and p is an integer of 1 to 3;
   R² to R⁴ are each independently hydrogen or a C₁₋₄ alkyl group;
   R⁵ is a C₁₋₄ alkyl group;
   R⁶ is a C₁₋₂ alkyl group;
   R⁷ is a C₁₋₇ alkyl group;
   n is an integer of 10 to 500;
   m is an integer of 1 to 5; and
   q is an integer of 0 to 2.
[10] The polymer-coated microparticles according to [8], wherein, in the general formula (1), R¹ is -(CH₂)ₚ-N₃, and p is an integer of 1 to 3;
   R² to R⁴ are each independently hydrogen or a C₁₋₄ alkyl group;
   R⁵ is a C₁₋₄ alkyl group;
   R⁶ is a C₁₋₂ alkyl group;
   R⁷ is a C₁₋₇ alkyl group;
   n is an integer of 10 to 500;
   m is an integer of 1 to 5; and
   q is an integer of 0 to 2.
[11] The polymer-coated microparticles according to [8], wherein, in the general formula (1), R¹ is -(CH₂)ₚ-NH₂, and p is an integer of 1 to 3;
   R² to R⁴ are each independently hydrogen or a C₁₋₄ alkyl group;
   R⁵ is a C₁₋₄ alkyl group;
   R⁶ is a C₁₋₂ alkyl group;
   R⁷ is a C₁₋₇ alkyl group;
   n is an integer of 10 to 500;
   m is an integer of 1 to 5; and
   q is an integer of 0 to 2.
[12] The polymer-coated microparticles according to [8], wherein, in the general formula (1), R¹ is -(CH₂)ₚ-COOH, and p is an integer of 1 to 3;
   R² to R⁴ are each independently hydrogen or a C₁₋₄ alkyl group;
   R⁵ is a C₁₋₄ alkyl group;
   R⁶ is a C₁₋₂ alkyl group;
   R⁷ is a C₁₋₇ alkyl group;
   n is an integer of 10 to 500;
   m is an integer of 1 to 5; and
   q is an integer of 0 to 2.
[13] The polymer-coated microparticles according to any one of [8] to [12], wherein the number-average molecular weight (Mn) of the oxyethylene chain-containing polymer is 500 to 100,000.
[14] The polymer-coated microparticles according to any one of [8] to [13], wherein the ratio of the weight-average molecular weight (Mw) of the oxyethylene chain-containing polymer to the number-average molecular weight (Mn) of the polymer is 1.0 or more and 3.0 or less.
[15] The polymer-coated microparticles according to any one of [8] to [14], wherein the microparticles are inorganic microparticles of at least one selected from the group consisting of gold, platinum, silver, and silicon dioxide.
[16] The polymer-coated microparticles according to any one of [8] to [15], wherein the average particle diameter of the microparticles is 10 nm or more and 500 nm or less.
[17] A cell-adhesive composition including the polymer-coated microparticles according to any one of [8] to [16].

### Advantageous Effects of Invention

The present invention can provide: a cell-adhesive composition including an oxyethylene chain-containing polymer having excellent biocompatibility and excellent reactivity; or microparticles coated with the polymer. In addition, the present invention can provide microparticles that have excellent biocompatibility and are coated with an oxyethylene chain-containing polymer. These cell-adhesive composition and polymer-coated microparticles have excellent biocompatibility and cell adhesiveness, can achieve high stealthiness, and thus, can be utilized as a DDS.

### BRIEF DESCRIPTION OF DRAWINGS

[FIG. 1] FIG. 1 is ¹H-NMR spectra of a reaction product (oxyethylene chain-containing polymer) and a precursor thereof that were subjected to measurement in Synthesis Example 1.
[FIG. 2] FIG. 2 is ¹H-NMR spectra of a reaction product (oxyethylene chain-containing polymer) and a precursor thereof that were subjected to measurement in Synthesis Example 2.

### DESCRIPTION OF EMBODIMENTS

### <Cell-adhesive Composition>

A cell-adhesive composition according to the present invention includes an oxyethylene chain-containing polymer detailed below. In addition, a cell-adhesive composition according to the present invention may include the polymer-coated microparticles detailed below. A cell-adhesive composition according to the present invention may include a drug or another additive besides the oxyethylene chain-containing polymer and the polymer-coated microparticles. The oxyethylene chain-containing polymer detailed below has excellent biocompatibility and still has excellent cell adhesiveness. Accordingly, the cell-adhesive composition including an oxyethylene chain-containing polymer has excellent biocompatibility and cell adhesiveness, can achieve high stealthiness, and thus, can be utilized as a DDS.

### <Oxyethylene Chain-containing Polymer>

An oxyethylene chain-containing polymer is represented by the following general formula (1).

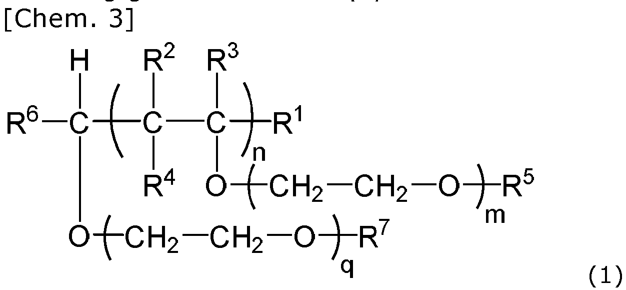

In the general formula (1), R¹ is -(CH₂)ₚ-SH, -(CH₂)ₚ-N₃, -(CH₂)ₚ-NH₂, or -(CH₂)ₚ-COOH.

p is an integer of 1 to 5, preferably an integer of 1 to 3, more preferably 1 or 2.

R² to R⁴ are each independently hydrogen or a C₁₋₁₀ alkyl group, preferably hydrogen or a C₁₋₄ alkyl group, more preferably hydrogen or a C₁₋₃ alkyl group, still more preferably hydrogen or a C₁₋₂ alkyl group, still more preferably hydrogen.

R⁵ is a C₁₋₁₀ alkyl group, preferably a C₁₋₁₀ alkyl group, more preferably a C₁₋₄ alkyl group, still more preferably a C₁₋₂ alkyl group.

R⁶ is hydrogen or a C₁₋₅ alkyl group, preferably a C₁₋₂ alkyl group, more preferably a methyl group.

R⁷ is a C₁₋₁₀ alkyl group, preferably a C₁₋₇ alkyl group, more preferably a C₁₋₄ alkyl group.

n is an integer of 5 to 1000, preferably an integer of 10 to 500, more preferably an integer of 20 to 400.

m is an integer of 1 to 10, preferably an integer of 1 to 5, more preferably an integer of 1 to 3.

q is an integer of 0 to 5, preferably an integer of 0 to 2, more preferably an integer of 0 to 1.

In this regard, the above-described alkyl group may be linear or branched.

The oxyethylene chain-containing polymer has a highly reactive functional group introduced into one end (ω end) thereof, in which the functional group is a thiol group, azido group, amino group, or carboxyl group. Thus, the polymer has excellent biocompatibility and still can modify a base material, microparticles, or the like. Because of this, the polymer can give biocompatibility to a base material, microparticles, or the like. In addition, such a highly reactive functional group can be further converted to further enhance capabilities such as reactivity and a modifying capability.

Examples of a preferable embodiment of an oxyethylene chain-containing polymer represented by the general formula (1) include the below-described polymers. In the following formula, n is an integer of 5 to 1000, preferably an integer of 10 to 500, more preferably an integer of 20 to 400.

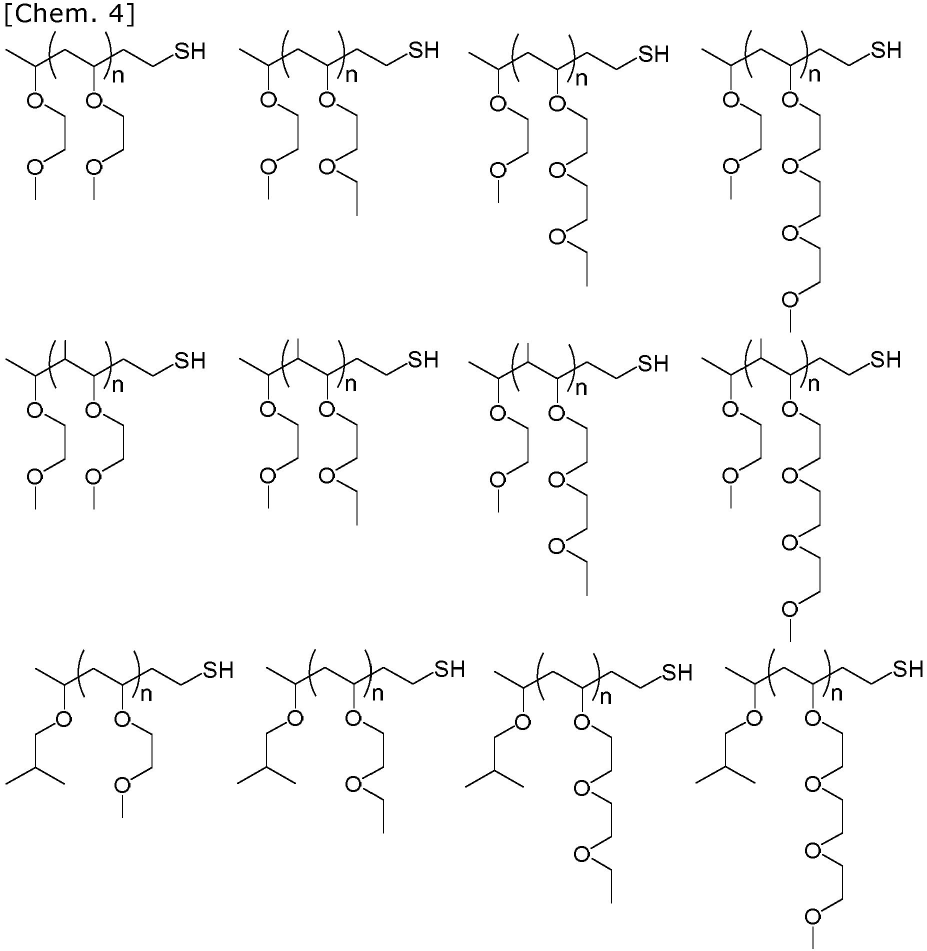

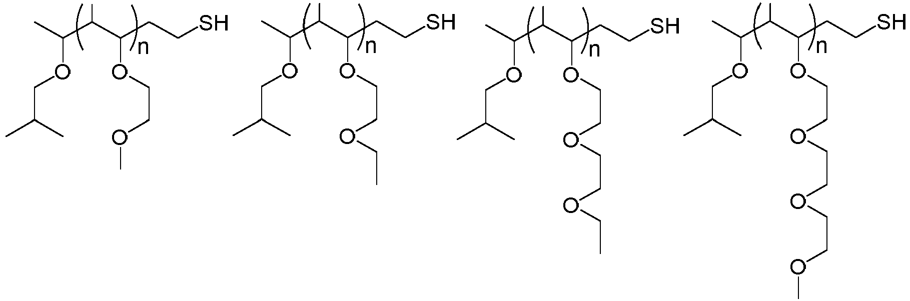

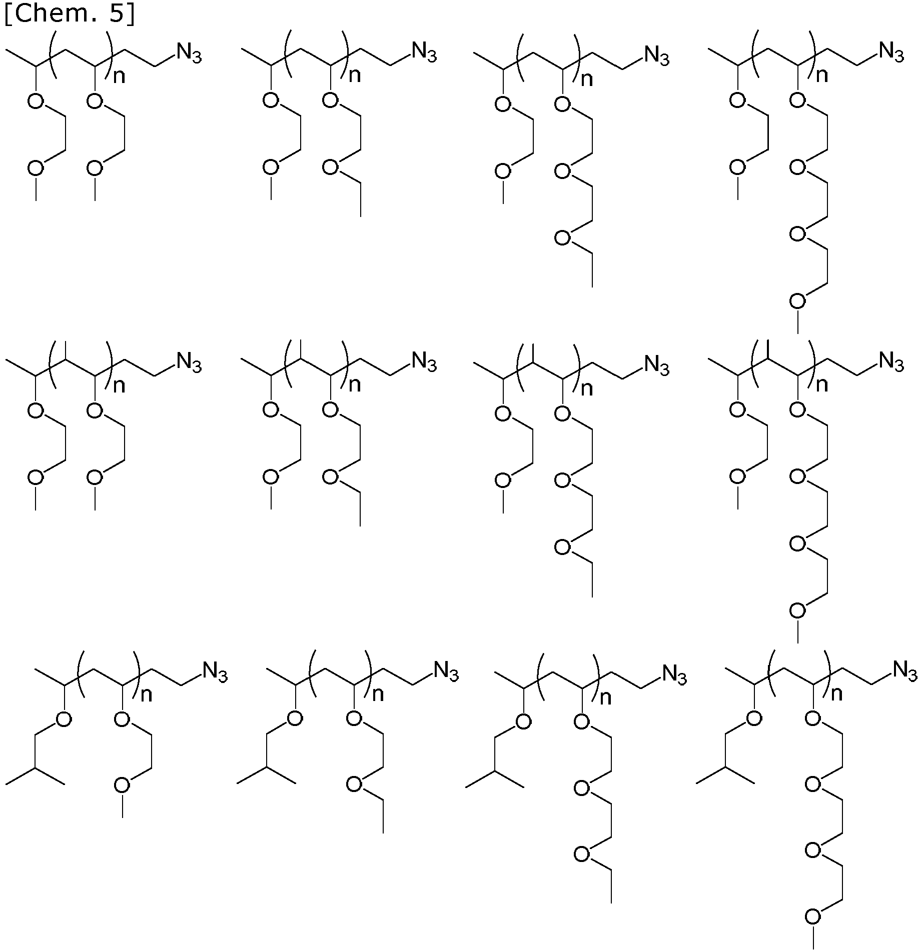

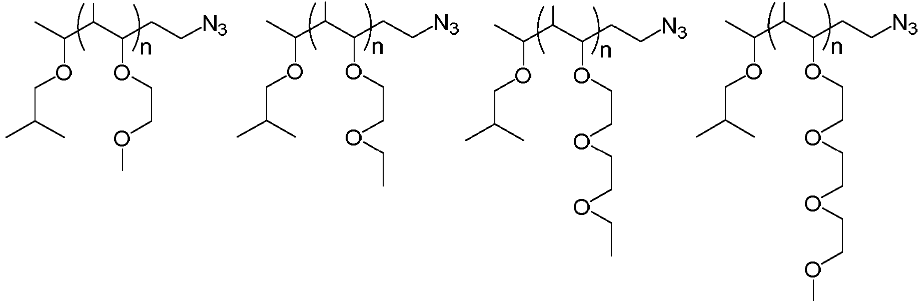

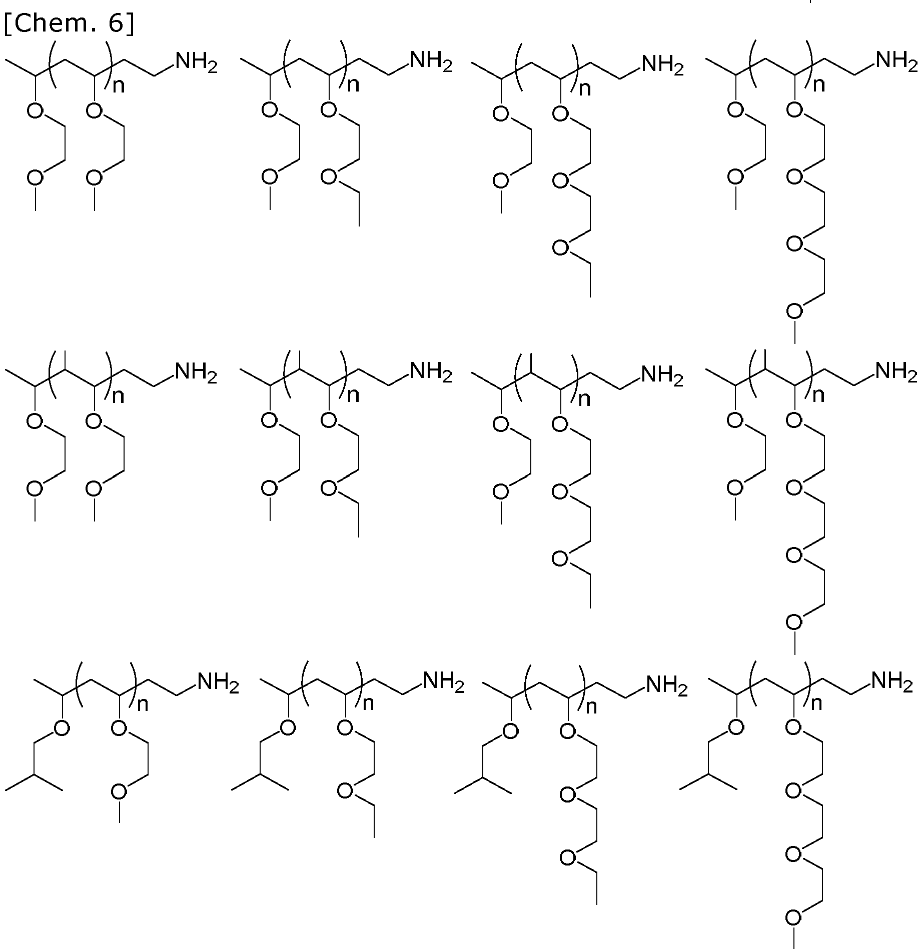

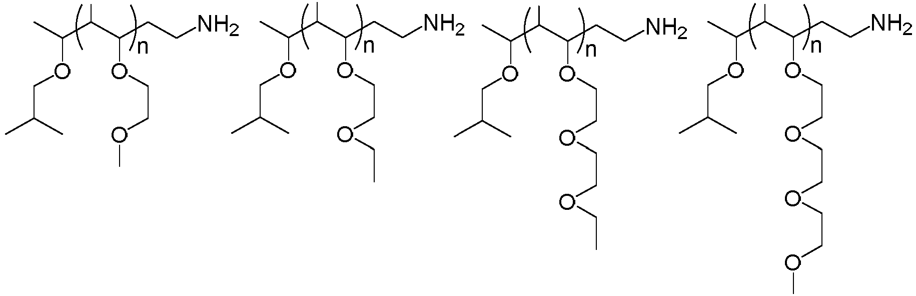

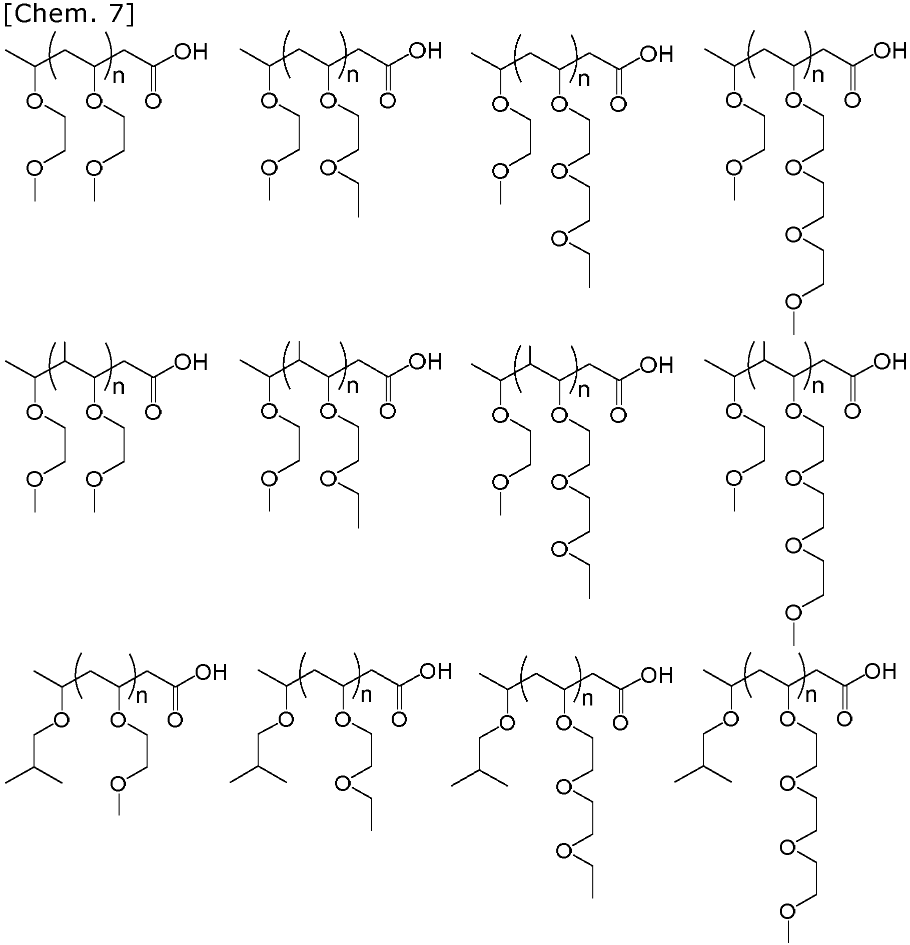

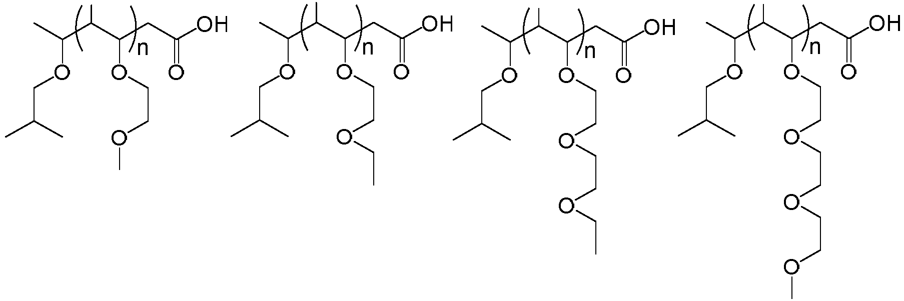

The number-average molecular weight (Mn) of the oxyethylene chain-containing polymer is preferably 500 or more and 100,000 or less. The lower limit of the number-average molecular weight (Mn) is more preferably 700 or more, still more preferably 1,000 or more, still more preferably 1,500 or more, and the upper limit is more preferably 70,000 or less, still more preferably 50,000 or less, still more preferably 30,000 or less, particularly preferably 20,000 or less, most preferably 10,000 or less. The oxyethylene chain-containing polymer having a number-average molecular weight (Mn) in the above-described ranges has excellent biocompatibility, and in addition, can more easily modify a base material, microparticles, and the like, and more easily give biocompatibility.

The ratio (the molecular-weight distribution, Mw/Mn) of the weight-average molecular weight (Mw) of the oxyethylene chain-containing polymer to the number-average molecular weight (Mn) of the polymer is preferably 1.0 or more and 3.0 or less, more preferably 1.0 or more and 2.0 or less, still more preferably 1.0 or more and 1.5 or less, still more preferably 1.0 or more and 1.3 or less. The oxyethylene chain-containing polymer having a molecular-weight distribution (Mw/Mn) in the above-described ranges makes it easier to keep the capability of the polymer uniformly.

In this regard, the number-average molecular weight (Mn) and the weight-average molecular weight (Mw) in the present invention can be calculated from a standard polystyrene calibration curve based on a gel permeation chromatography (GPC) method.

### <Method of Producing Oxyethylene Chain-containing Polymer>

Embodiments of a method of producing the oxyethylene chain-containing polymer will be described. First, using a vinyl ether as a raw-material monomer, for example, an oxyethylene chain-containing polymer having a carbonyl group at one end thereof and represented by the following general formula (2) is synthesized through living cationic polymerization in the presence of a polymerization initiator. Upon completion of the polymerization, a small amount of water is added, so that the end cation reacts with the water, thus enabling a carbonyl group to be introduced. Subsequently, the carbonyl group at one end of the oxyethylene chain-containing polymer represented by the following general formula (2) is converted to thereby enable a reactive functional group (thiol group, azido group, amino group, or carboxyl group) to be introduced.

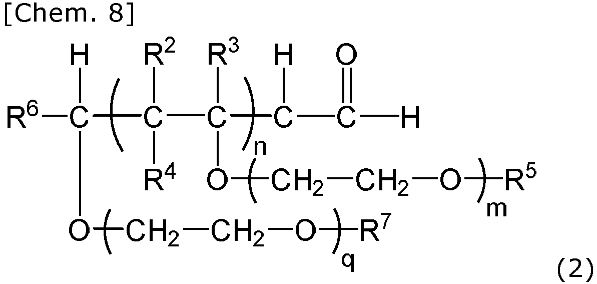

In this regard, R² to R⁷, n, m, and q in the general formula (2) are the same as R² to R⁷, n, m, and q in the general formula (1).

In addition, preferable aspects of R² to R⁷, n, m, and q in the general formula (2) are the same as preferable aspects of R² to R⁷, n, m, and q in the general formula (1).

A vinyl ether usable as a raw-material monomer is, for example, a vinyl ether represented by the following general formula (3).

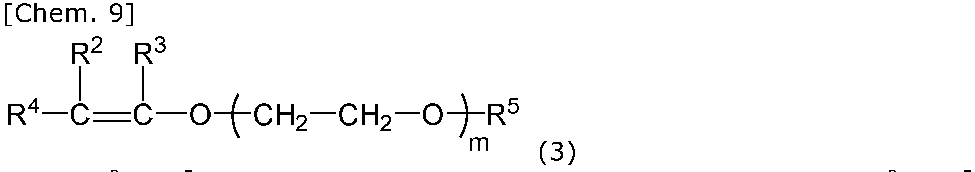

R² to R⁵ and m in the general formula (3) are the same as R² to R⁵ and m in the general formula (1).

In addition, preferable aspects of R² to R⁵ and m in the general formula (2) are the same as preferable aspects of R² to R⁵ and m in the general formula (1).

Examples of a vinyl ether represented by the general formula (3) include 2-methoxyethylvinyl ether (MOVE), 2-ethoxyethylvinyl ether (EOVE), diethylene glycol monoethylmonovinyl ether (EOEOVE), and triethylene glycol monomethylvinyl ether (TEGMVE).

A polymerization initiator to be used in the polymerization of an oxyethylene chain-containing polymer is not particularly limited, subject to being a polymerization initiator that advances cationic polymerization in a living manner. The initiator may be a conventionally known polymerization initiator. Examples of a polymerization initiator to be suitably used, for example, a living cationic polymerization initiator for vinyl ethers, include HI/I₂-based initiators (for example, in JP S60-228509A) and polymerization initiators (for example, in JP3096494B2, JP H07-002805B2, JP S62-257910A, JP H01-108202A, and JP H01-108203A) obtained by combining a Lewis-acid catalyst (organic aluminum compound or the like) with an additive (ether or the like) such as a base.

The usage of the polymerization initiator is preferably 0.001 to 20 mol%, more preferably 0.01 to 10 mol%, particularly preferably 1 mol% or less, with respect to the total amount of the raw-material monomers.

In addition, the living cationic polymerization reaction is preferably performed in the presence of, or may be performed in the absence of, a suitable organic solvent. Examples of an organic solvent that is usable include aromatic hydrocarbon solvents such as benzene, toluene, and xylene; aliphatic hydrocarbon solvents such as propane, n-butane, isobutane, n-pentane, isopentane, n-hexane, n-heptane, n-octane, isooctane, decane, hexadecane, and cyclohexane; halogenated hydrocarbon solvents such as methylene chloride, ethylene chloride, and carbon tetrachloride; and ether solvents such as diethyl ether, dibutyl ether, tetrahydrofuran (THF), dioxane, and ethylene glycol diethyl ether. These organic solvents may be used singly or in combination of two or more kinds thereof, as necessary. In addition, among these organic solvents, hydrocarbon solvents such as aromatic hydrocarbon solvents and aliphatic hydrocarbon solvents are preferable, and toluene, THF, and the like are preferable.

The polymerization temperature in the living cationic polymerization reaction depends, for example, on the kinds of a polymerization initiator, monomer, and solvent that are used, and is usually -80 to 150°C, preferably -50 to 100°C, particularly preferably -20 to 80°C. In addition, the polymerization time depends, for example, on the polymerization initiator, monomer, and solvent that are used and on the reaction temperature, and is usually approximately 10 minutes to 100 hours. The polymerization reaction may be suitably performed using any of a batch method and a continuous method. After the polymerization reaction, a purification treatment, if desired, may be performed using a known method in order to remove an unreacted monomer.

Below, one example of polymerization reaction of an oxyethylene chain-containing polymer (PMOVE) is illustrated, in which 2-methoxyethylvinyl ether (MOVE) is used as a raw-material monomer. First, a PMOVE having a carbonyl group at one end thereof is synthesized from an MOVE in the presence of a polymerization initiator in accordance with the following reaction formula (I). Upon completion of the polymerization, a small amount of water is added, so that the end cation reacts with the water, thus enabling a carbonyl group to be introduced. In respect of the polymerization reaction conditions and the like for the following reaction formula (I), J. Polym. Sci., Part A: Polym. Chem. 2005, 43, 468-472 can be referred to. In the following reaction formulae (I) to (V), n in PMOVE is an integer of 5 to 1000, preferably an integer of 10 to 500, more preferably an integer of 20 to 400, as in the general formula (1).

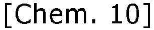

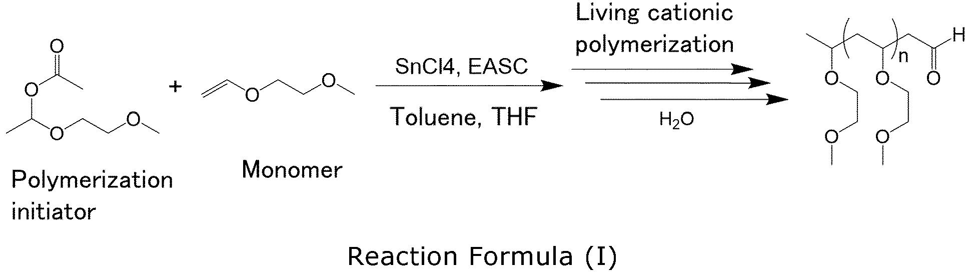

Below, a method (a reaction formula) of synthesizing a PMOVE will be described in respect of each kind of reactive functional group. A catalyst, polymerization initiator, solvent, and the like in the following series of reactions may be suitably selected with reference to a conventional known method. In addition, the polymerization reaction conditions such as a polymerization temperature and a polymerization time may be set suitably.

In a case where the reactive functional group is a thiol group, the carbonyl group at one end of the PMOVE obtained as described above can be converted in accordance with the following reaction formula (II) to synthesize a PMOVE having a thiol group at one end thereof. In respect of the polymerization reaction conditions and the like for the following reaction formula (II), Journal of Colloid and Interface Science, 428 (2014), 276-285 can be referred to. In the following reaction formula (II), EtOH represents ethanol, MsCl represents methanesulfonyl chloride, TEA represents triethylamine, KSAc represents potassium thioacetate, DMSO represents dimethyl sulfoxide, MeOH represents methanol, and NaOH represents sodium hydroxide.

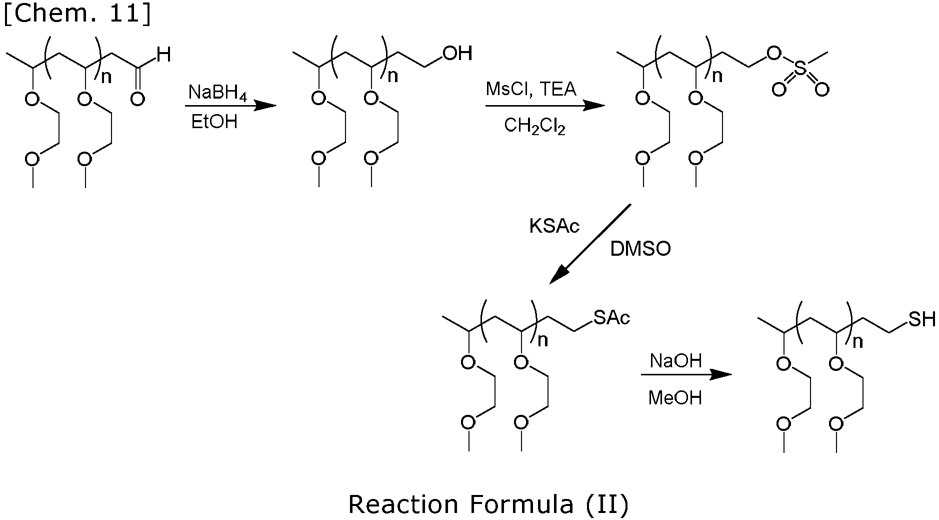

In a case where the reactive functional group is an azido group, the carbonyl group at one end of the PMOVE obtained as described above can be converted in accordance with the following reaction formula (III) to synthesize a PMOVE having an azido group at one end thereof. In respect of the polymerization reaction conditions and the like for the following reaction formula (III), Med Chem Res (2016) 25: 824-833 can be referred to. In the following reaction formula (III), NaN₃ is sodium azide, and the other abbreviations are the same as the abbreviations in the above-described reaction formula (II).

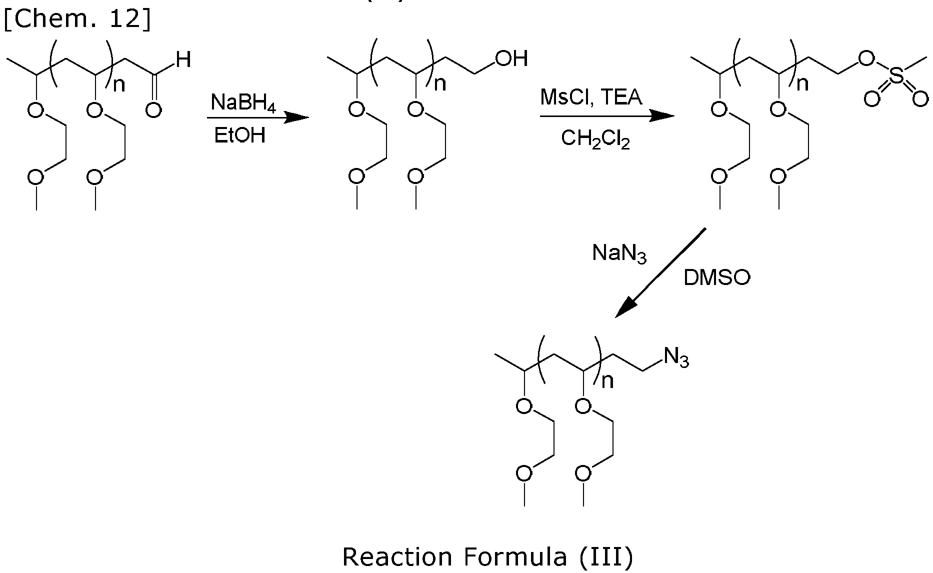

In a case where the reactive functional group is an amino group, the carbonyl group at one end of the PMOVE obtained as described above can be converted in accordance with the following reaction formula (IV) to synthesize a PMOVE having an amino group at one end thereof. In respect of the polymerization reaction conditions and the like for the following reaction formula (IV), Med Chem Res (2016) 25: 824-833 can be referred to. In the following reaction formula (IV), PPh₃ is triphenylphosphine, and the other abbreviations are the same as the abbreviations in the above-described reaction formula (II).

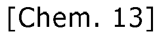

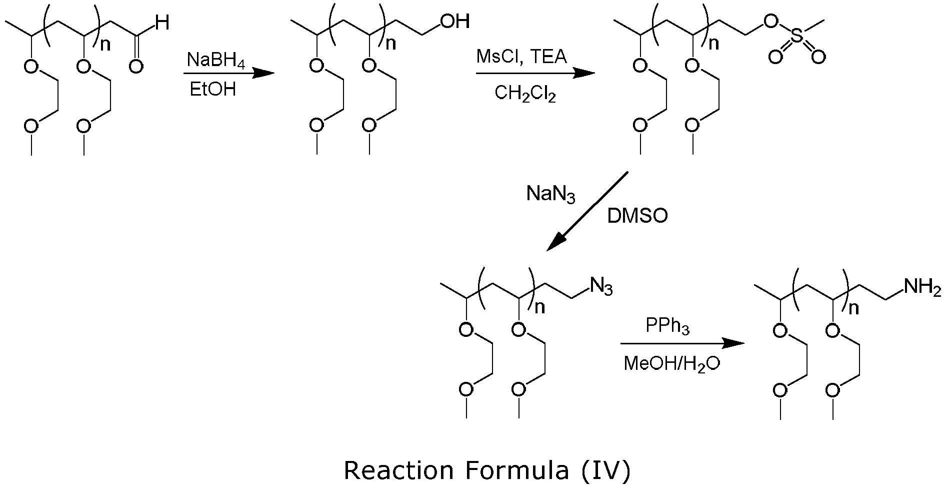

In a case where the reactive functional group is a carboxyl group, the carbonyl group at one end of the PMOVE obtained as described above can be converted in accordance with the following reaction formula (IV) to synthesize a PMOVE having a carboxyl group at one end thereof. In the following reaction formula (V), NaClO₂ represents sodium chlorite, and NaH₂PO₄ represents sodium dihydrogenphosphate.

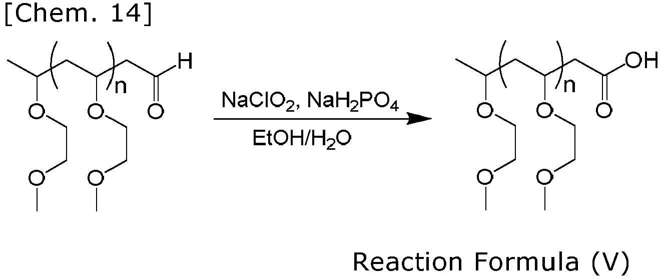

### <Drug>

A cell-adhesive composition according to the present invention may further contain a conventional known drug. Binding the oxyethylene chain-containing polymer to the drug enables the drug to have higher biocompatibility and cell adhesiveness, and be utilized as a DDS. Examples of the drug include, but are not particularly limited to, nucleic acid therapeutics, anticancer drugs, and anti-inflammatory drugs.

Examples of nucleic acid therapeutics include an siRNA, plasmid DNA, and mRNA.

Examples of the anticancer drugs include BCG, actinomycin D, asparaginase, aceglatone, anastrozole, allopurinol, anthracycline, bicalutamide, antiandrogen, idarubicin, ifosfamide, imatinib, irinotecan, interferon, interferon-alpha, interleukin-2, ubenimex, exemestane, estramustine, estrogen, etoposide, enocitabine, epirubicin, oxaliplatin, octreotide, capecitabine, carboquone, carboplatin, carmofur, cladribine, clarithromycin, Krestin, ketoconazole, gefitinib, gemcitabine, gemtuzumab, goserelin, cyclophosphamide, cisplatin, sizofiran, cytarabine, cyproheptadine, zinostatin stimalamer, cetuximab, sobuzoxane, tamoxifen, daunorubicin, dacarbazine, dactinomycin, thiotepa, tegafur, tegafur/uracil, tegafur/gimeracil/oteracil, dexametasone, topotecan, trastuzumab, triptorelin, tretinoin, toremifene, doxifluridine, doxorubicin, docetaxel, nimustine, neocarzinostatin, nedaplatin, paclitaxel, hydroxyurea, hydroxycarbamide, bicalutamide, vinorelbine, vincristine, vindesine, vinblastine, picibanil, pirarubicin, fadrozole, fluorouracil, flutamide, fludarabine, busulfan, bleomycin, prednisone, procarbazine, progestin, peplomycin, pentostatin, porfimer sodium, mitomycin, mitoxantrone, mitotane, mesna, methotrexate, medroxyprogesterone, mercaptopurine, melphalan, ranimustine, rituximab, leuprolide, retinoic acid, lentinan, and leucovorin. These anticancer drugs may be used singly, or may be used in combination of two or more kinds thereof.

Examples of the anti-inflammatory drugs include steroidal anti-inflammatory drugs and non-steroidal anti-inflammatory drugs.

Examples of the steroidal anti-inflammatory drugs include corticosteroid-based anti-inflammatory drugs, examples of which include dexametasone, triamcinolone acetonide, beclometasone, hydrocortisone, methyl prednisolone, prednisolone, prednisone, triamcinolone diacetate, cortisone, cortisol, parametasone, triamcinolone, diflucortolone, difluprednate, diflorasone, flumetasone, fluocinonide, fluocinolone acetonide, alclometasone, fludrocortisone, and salts thereof. More specific examples include dexametasone, triamcinolone acetonide, beclometasone dipropionate, hydrocortisone succinate, methylprednisolone sodium succinate, dexametasone acetate, hydrocortisone acetate, prednisolone acetate, dexametasone methasulfobenzoic acid, triamcinolone diacetate, prednisolone butylacetate, dexametasone phosphate, hydrocortisone phosphate, prednisolone phosphate, betametasone phosphate, prednisolone succinate, cortisone acetate, parametasone acetate, methylprednisolone acetate, triamcinolone, hydrocortisone, prednisolone, betametasone, prednisolone valerate, diflucortolone valerate, dexametasone valerate, betametasone valerate, difluprednate acetate, diflorasone acetate, difluprednate, betametasone dipropionate, flumetasone pivalate, fluocinonide, fluocinolone acetonide, alclometasone dipropionate, beclometasone dipropionate, clobetasone butyrate, hydrocortisone butyrate, hydrocortisone butyrate propionate, fludrocortisone butyrate, dexametasone palmitate, and methyl prednisolone.

Examples of the non-steroidal anti-inflammatory drugs include NSAID and COX-2 inhibitors. More specific examples include acetylsalicylic acid, alclofenac, alminoprofen, benoxaprofen, butibufen, bucloxic acid, carprofen, celecoxib, clidenac, diclofenac, diflunisal, etodolac, fenbufen, fenoprofen, fentiazic, flufenamic acid, flufenasol, flurbiprofen, furofenac, ibufenac, ibuprofen, indomethacin, indoprofen, isoxepac, isoxicam, ketoprofen, ketorolac, meclofenamic acid, mefenamic acid, meloxicam, miroprofen, naproxen, oxaprozin, oxyphenbutazone, oxpinac, parecoxib, phenylbutazone, piclamilast, piroxicam, pirprofen, pranoprofen, rofecoxib, sudoxicam, sulindac, suprofen, tenclofenac, tiaprofenic acid, tolfenamic acid, tolmetin, tramadol, valdecoxib, zomepirac, and salts thereof.

### <Polymer-coated Microparticles>

Polymer-coated microparticles according to the present invention are microparticles coated with the above-described PMOVE. Such polymer-coated microparticles have excellent biocompatibility and cell adhesiveness, can achieve high stealthiness, and thus, can be utilized as a DDS.

As microparticles coated with the above-described PMOVE, any of inorganic microparticles and organic microparticles can be used. Examples of the inorganic microparticles include, but are not particularly limited to, microparticles of gold, platinum, silver, or silicon dioxide. In addition, examples of the organic microparticle include, but are not particularly limited to, polystyrene, polymethyl acrylate, polymethyl methacrylate, polyacrylamide, and copolymers thereof.

The average particle diameter of the microparticles coated with the above-described PMOVE is, but not particularly limited to, preferably 10 nm or more and 500 nm or less, more preferably 50 nm or more and 300 nm or less, still more preferably 70 nm or more and 200 nm or less, still more preferably 100 nm or more and 150 nm or less. The microparticles having an average particle diameter in the above-described ranges are more easily utilized as a DDS.

In this regard, the average particle diameter of the microparticles can be measured by dynamic light scattering.

### EXAMPLES

Next, the present invention will be described in more detail with reference to Examples and Comparative Examples. The present invention should not be restricted to these Examples or the like in any way.

In Synthesis Examples, the structure of a polymer was determined from the results of ¹H-NMR analysis. In addition, the number-average molecular weight (Mn) and molecular-weight distribution (Mw/Mn) of a polymer were determined from the results of GPC analysis of the molecular weight (in terms of polystyrene). The analytical device, measurement conditions, and the like in each measurement are described below.

### (Measurement Conditions for NMR)

- Device: Model No. ECS-400, manufactured by JEOL Ltd.
- Solvent: deuteriochloroform
- Measurement temperature: 25°C

### (Measurement Conditions for GPC)

- Device: "HLC-8320GPC", manufactured by Tosoh Corporation
- Detector: an RI detector
- Mobile phase: tetrahydrofuran
- Flow rate: 1 mL/min
- Column: "Shodex LF-804" × 3 columns, manufactured by Showa Denko K.K.
- Column temperature: 40°C

### [Synthesis Example 1]

### Synthesis of oxyethylene chain-containing polymer having thiol group at one end (PMOVE-SH) (reaction formulae (I) + (II))

Using 2-methoxyethylvinyl ether (MOVE) as a raw-material monomer, a PMOVE having a carbonyl group at one end thereof (PMOVE-CHO) was obtained in accordance with the above-described reaction formula (I). Specifically, 5.8 mL of MOVE as a raw-material monomer, 147 µL of MOEA, namely an acetic acid adduct of MOVE, as a polymerization initiator, 90 mL of toluene, 1.6 mL of THF, 108 µL of 2,6-di-tert-butylpyridine, 2.2 mL of ethyl aluminum sesquichloride (25% in a toluene solution), and 1.0 mL of tin chloride (IV) (1.0 M in a heptane solution) were mixed, and allowed to undergo polymerization reaction. Upon completion of the polymerization, 1.0 mL of water was mixed, and a carbonyl group was introduced into one end. The solvent was removed using an evaporator, the residue was redissolved in ethanol, and then purified using a 0.45 µm syringe filter. The solvent was again removed to give a PMOVE-CHO.

Herein, a polymer having a functional group at one end is referred to as a "polymer name-functional group name".

Then, the carbonyl group at one end of PMOVE was converted into a thiol group in accordance with the above-described reaction formula (II). Specifically, 1.0 g of PMOVE-CHO, 0.3 g of sodium borohydride, and 20 mL of ethanol were first mixed, and the resulting mixture was stirred overnight. The resulting mixture was dissolved in water, and dialytically purified through a dialysis membrane having an MWCO of 3,500 to give a PMOVE-OH. Subsequently, 500 mg of PMOVE-OH, 12 mL of CH₂Cl₂, 63 µL of TEA, and 23 µL of MsCl were mixed, allowed to react under argon at 0°C for 0.5 hours, and further allowed to react at room temperature for 3.5 hours. The resulting solution was washed with water and saturated saline, and the solvent was removed with an evaporator to give a PMOVE-Ms. Subsequently, 500 mg of PMOVE-Ms, 12 mL of dehydrated DMSO, and 57 mg of KSAc were mixed and allowed to react under argon at 40°C for 18 hours. The resulting solution was quenched with 1.0 M hydrochloric acid, and then, the solvent was removed. The resulting product was redissolved in water, and dialytically purified through a dialysis membrane having an MWCO of 3,500 to give a PMOVE-SAc. Finally, 500 mg of PMOVE-SAc and 12 mL of dehydrated methanol were mixed and sparged with argon. To the resulting solution, 20 mg of NaOH and 46 mg of dithiothreitol were mixed, and allowed to react for 1 hour. The resulting solution was quenched with 1.0 M hydrochloric acid, and the resulting solution was dialytically purified through a dialysis membrane having an MWCO of 3,500 at 4°C in a refrigerator to give a PMOVE-SH.

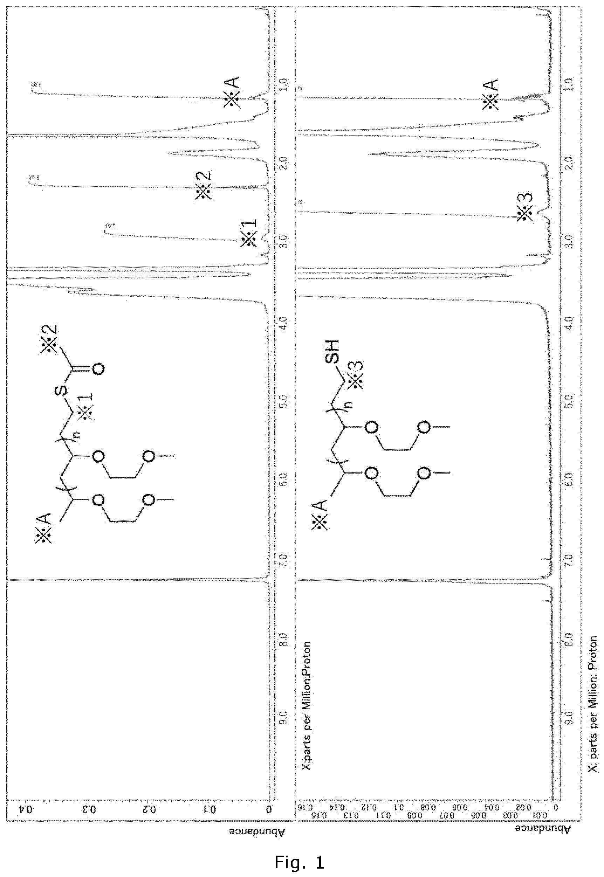

The resulting reaction product (PMOVE-SH) and a precursor thereof (PMOVE-SAc) were subjected to a ¹H-NMR measurement under the above-described conditions to give ¹H-NMR spectra illustrated in FIG. 1. The upper diagram in FIG. 1 is the spectrum of the precursor, and the lower diagram in FIG. 1 is the spectrum of the reaction product. The spectra were analyzed with the results as follows: in the spectrum of the precursor, the peak (*1 in the upper diagram in FIG. 1) derived from the methylene group (CH₂) adjacent to the thioacetyl group (SCOCH₃) was observed at the position of 2.90 ppm; and in the spectrum of the reaction product, it was observed that the peak (*3 in the lower diagram in FIG. 1) derived from the methylene group adjacent to the thiol group shifted to the position of 2.60 ppm. Additionally, in the spectrum of the precursor, the peak (*2 in the upper diagram in FIG. 1) derived from the thioacetyl group was observed at the position of 2.30 ppm, but in the spectrum of the reaction product, the peak derived from the thioacetyl group had vanished. These results have verified that a thiol group was introduced into one end of the PMOVE. Additionally, compared with the value of integral of the peak (*A) derived from the methyl group of the α end, the ratio of introduction of a thiol group into one end was approximately 100%.

The number-average molecular weight (Mn) and molecular-weight distribution (Mw/Mn) of the PMOVE-SH obtained as described above were measured with the results as follows: Mn = 5,000; and Mw/Mn = 1.05.

### [Synthesis Example 2]

### Synthesis of oxyethylene chain-containing polymer having azido group at one end (PMOVE-N₃) (reaction formulae (I) + (III))

A PMOVE-CHO was obtained in the same manner as in Synthesis Example 1. Then, the carbonyl group at one end of PMOVE was converted into an azido group in accordance with the above-described reaction formula (III). Specifically, 500 mg of PMOVE-Ms, 12 mL of dehydrated DMSO, and 39 mg of NaN₃ were mixed and allowed to react under argon at 70°C for 18 hours. The resulting solution was quenched with water, and then, the solvent was removed. The resulting product was redissolved in water, and dialytically purified through a dialysis membrane having an MWCO of 3,500 to give a PMOVE-N₃.

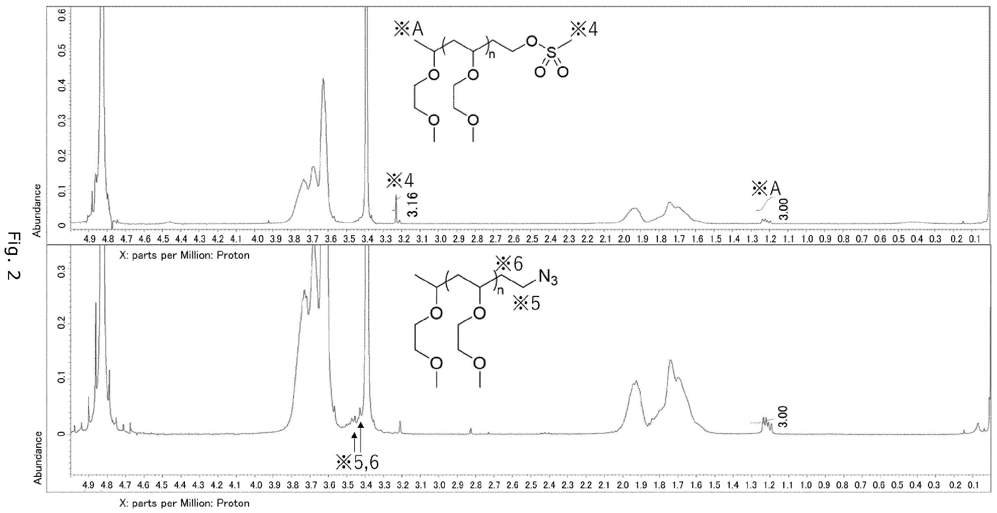

The resulting reaction product (PMOVE-N₃) and a precursor thereof (PMOVE-Ms) were subjected to a ¹H-NMR measurement under the above-described conditions to give ¹H-NMR spectra illustrated in FIG. 2. The upper diagram in FIG. 2 is the spectrum of the precursor, and the lower diagram in FIG. 2 is the spectrum of the reaction product. The spectra were analyzed with the results as follows: in the spectrum of the precursor, the peak (*4 in the upper diagram in FIG. 2) derived from the methyl group (CH₃) in the methylsulfonyl group (CH₃SO₂) was observed at the position of 3.24 ppm, but in the spectrum of the reaction product, the peak derived from the methyl group in the methylsulfonyl group had vanished. Additionally, in the spectrum of the reaction product, the peaks (*5 and *6 in the lower diagram in FIG. 2) derived from the ethylene group (CH₂CH₂) adjacent to the azido group were observed at the positions between 3.40 and 3.50 ppm. These results have verified that an azido group was introduced into one end of the PMOVE. In addition, the value of integral of the peak (*A) derived from the methyl group of the a end was calculated, with the result that the ratio of introduction of a methylsulfonyl group into one end of the precursor was approximately 100%.

The number-average molecular weight (Mn) and molecular-weight distribution (Mw/Mn) of the PMOVE-N₃ obtained as described above were measured with the results as follows: Mn = 5,000; and Mw/Mn = 1.05.

### [Synthesis Example 3]

### Synthesis of oxyethylene chain-containing polymer having amino group at one end (PMOVE-NH₂) (reaction formulae (I) + (IV))

A PMOVE-CHO was obtained in the same manner as in Synthesis Example 1. Then, the carbonyl group at one end of PMOVE was converted into an amino group in accordance with the above-described reaction formula (IV). Specifically, 500 mg of PMOVE-N₃, 525 mg of PPh₃, 8.7 mL of MeOH, and 3.3 mL of water were mixed, and allowed to react for 5 hours under reflux at 90°C. The resulting solution was quenched with 1.0 M hydrochloric acid, and then, the solvent was removed. The resulting product was redissolved in water, and dialytically purified through a dialysis membrane having an MWCO of 3,500 to give a PMOVE-NH₂.

The PMOVE-NH₂ obtained as described above was quantitated through a cation-exchange column (SP-5PW), using GPC (an RI detector: RI-2031 Plus (manufactured by JASCO Corporation); a GPC pump: PU-1580 (manufactured by JASCO Corporation; mobile phase, an aqueous solution of 2 mM NaCl). The ratio of introduction of an amino group into one end was approximately 100%. Additionally, from this result, it is estimated that the ratio of introduction of an azido group into one end was approximately 100% also in the polymer having an azido group at one end in Synthesis Example 2, in which the polymer was a precursor of the polymer having an amino group at one end in Synthesis Example 3.

The number-average molecular weight (Mn) and molecular-weight distribution (Mw/Mn) of the PMOVE-NH₂ obtained as described above were measured with the results as follows: Mn = 5,000; and Mw/Mn = 1.05.

### [Synthesis Example 4]

### Synthesis of oxyethylene chain-containing polymer having carboxyl group at one end (PMOVE-COOH) (reaction formulae (I) + (V))

A PMOVE-CHO was obtained in the same manner as in Synthesis Example 1. Then, the carbonyl group at one end of PMOVE was converted into a carboxyl group in accordance with the above-described reaction formula (V). Specifically, 500 mg of PMOVE-CHO, 5.5 mL of ethanol, and 0.2 mL of 2-methyl-2-butene were mixed, and cooled to 0°C with good stirring. Subsequently, 34 mg of NaClO₂, 36 mg of NaH₂PO₄, and 0.9 mL of water were mixed to another flask, and stirred well. This aqueous solution was dropped into the ethanol solution, stirred for 5 minutes, and then further allowed to react at room temperature for 15 hours. The resulting solution was quenched with saturated saline, and the resulting solution was dialytically purified through a dialysis membrane having an MWCO of 3,500 to give a PMOVE-COOH.

The PMOVE-COOH obtained as described above was subjected to measurement by ¹H-NMR, verifying that a carboxyl group had been introduced into one end. In addition, the ratio of introduction of a carboxyl group into one end was approximately 100%.

The number-average molecular weight (Mn) and molecular-weight distribution (Mw/Mn) of the PMOVE-COOH obtained as described above were measured with the results as follows: Mn = 5,000; and Mw/Mn = 1.05.

### [Synthesis Example 5]

### Synthesis of oxyethylene chain-containing polymer having thiol group at one end (PMOVE-SH) (reaction formulae (I) + (II))

A PMOVE-SH was obtained in the same manner as in Synthesis Example 1 except that 3.5 mL of MOVE, 230 µL of MOEA, 138 mL of toluene, 2.4 mL of THF, 162 µL of 2,6-di-tert-butylpyridine, 3.3 mL of ethyl aluminum sesquichloride (25% in a toluene solution), and 1.5 mL of tin chloride (IV) (1.0 M in a heptane solution) were mixed.

The number-average molecular weight (Mn) and molecular-weight distribution (Mw/Mn) of the PMOVE-SH obtained as described above were measured with the results as follows: Mn = 2,000; and Mw/Mn = 1.06.

### [Synthesis Example 6]

### Synthesis of oxyethylene chain-containing polymer having thiol group at one end (PEOVE-SH) (reaction formulae (I) + (II))

A PEOVE-SH was obtained in the same manner as in Synthesis Example 1 except that 6.5 mL of 2-ethoxyethylvinyl ether (EOVE) as a raw-material monomer and 203 µL of EOEA namely an acetic acid adduct of EOVE as an initiator were mixed.

The number-average molecular weight (Mn) and molecular-weight distribution (Mw/Mn) of the PEOVE-SH obtained as described above were measured with the results as follows: Mn = 5,000; and Mw/Mn = 1.08.

### [Synthesis Example 7]

### Synthesis of oxyethylene chain-containing polymer having thiol group at one end (PEOEOVE-SH) (reaction formulae (I) + (II))

A PEOEOVE-SH was obtained in the same manner as in Synthesis Example 1 except that 8.7 mL of 2-(2-ethoxy)ethoxyethylvinyl ether (EOEOVE) as a raw-material monomer and 333 µL of EOEOEA namely an acetic acid adduct of EOEOVE as an initiator were mixed.

The number-average molecular weight (Mn) and molecular-weight distribution (Mw/Mn) of the PEOEOVE-SH obtained as described above were measured with the results as follows: Mn = 5,000; and Mw/Mn = 1.06.

### [Synthesis Example 8]

### Synthesis of oxyethylene chain-containing polymer having thiol group at one end (PTEGMVE-SH) (reaction formulae (I) + (II))

A PTEGMVE-SH was obtained in the same manner as in Synthesis Example 1 except that 9.6 mL of triethylene glycol monomethylvinyl ether (TEGMVE) as a raw-material monomer and 450 µL of TEGMEA namely an acetic acid adduct of TEGMVE as an initiator were mixed.

The number-average molecular weight (Mn) and molecular-weight distribution (Mw/Mn) of the PTEGMVE-SH obtained as described above were measured with the results as follows: Mn = 5,000; and Mw/Mn = 1.08.

### [Synthesis Example 9]

### Synthesis of oxyethylene chain-containing polymer having carbonyl group at one end (PMOVE-CHO) (reaction formula (I))

A PMOVE-CHO was obtained in the same manner as in Synthesis Example 1.

The number-average molecular weight (Mn) and molecular-weight distribution (Mw/Mn) of the PMOVE-CHO obtained as described above were measured with the results as follows: Mn = 5,000; and Mw/Mn = 1.05.

### [Synthesis Example 10]

### Synthesis of poly(n-butylvinyl ether) having thiol group at one end (PNBVE-SH)

A PNBVE-SH was obtained in the same manner as in Synthesis Example 1 except that n-butylvinyl ether (NBVE) as a raw-material monomer was used in place of 2-methoxyethylvinyl ether (MOVE).

The number-average molecular weight (Mn) and molecular-weight distribution (Mw/Mn) of the PNBVE-SH obtained as described above were measured with the results as follows: Mn = 5,000; and Mw/Mn = 1.10.

### [Example 1]

Using the PMOVE-SH obtained in Synthesis Example 1, gold microparticles having an average particle diameter of 150 nm were modified to give gold microparticles coated with PMOVE (PMOVE-coated gold microparticles). Specifically, the following test was performed.

### Testing method:

A dispersion of 150 nm gold microparticles modified with citric acid was concentrated by centrifugation, and then dispersed in 90 µL of water. Subsequently, 10 µL of the PMOVE-SH prepared at a concentration such that the average density of the polymer modification would become 1.12 pcs/nm² to gold microparticles was mixed. The resulting mixture was allowed to react at 60°C for 1 hour. After the reaction, the resulting solution was concentrated by centrifugation, and the supernatant liquid was removed. Then, the resulting solution was washed several times with water containing 0.25 mM sodium citrate and 0.05 v/v% Tween 20.

The average particle diameter of the PMOVE-coated gold microparticles obtained in the above-described test was measured by dynamic light scattering using a particle diameter measurement device (Model No.: Zetasizer Nano ZS, manufactured by Malvern Panalytical Ltd.). The average particle diameter was found to have been increased to 175.6 nm. From this result, it is estimated that the surfaces of the gold microparticles were successfully modified with the PMOVE.

### [Example 2]

Gold microparticles coated with the PMOVE (PMOVE-coated gold microparticles) were obtained in the same manner as in Example 1 except that the PMOVE-SH obtained in Synthesis Example 5 were used.

The average particle diameter of the resulting PMOVE-coated gold microparticles was found to have been increased to 161.0 nm. From this result, it is estimated that the surfaces of the gold microparticles were successfully modified with the PMOVE.

### [Example 3]

Gold microparticles coated with the PEOVE (PEOVE-coated gold microparticles) were obtained in the same manner as in Example 1 except that the PEOVE-SH obtained in Synthesis Example 6 were used.

The average particle diameter of the resulting PEOVE-coated gold microparticles was found to have been increased to 166.4 nm. From this result, it is estimated that the surfaces of the gold microparticles were successfully modified with the PEOVE.

### [Example 4]

Gold microparticles coated with the PEOEOVE (PEOEOVE-coated gold microparticles) were obtained in the same manner as in Example 1, except that the PEOEOVE-SH obtained in Synthesis Example 7 was used.

The average particle diameter of the resulting PEOEOVE-coated gold microparticles was found to have been increased to 166.9 nm. From this result, it is estimated that the surfaces of the gold microparticles were successfully modified with the PEOEOVE.

### [Example 5]

Gold microparticles coated with the PTEGMVE (PTEGMVE-coated gold microparticles) were obtained in the same manner as in Example 1 except that the PTEGMVE-SH obtained in Synthesis Example 8 was used.

The average particle diameter of the resulting PTEGMVE-coated gold microparticles was found to have been increased to 164.9 nm. From this result, it is estimated that the surfaces of the gold microparticles were successfully modified with the PTEGMVE.

### [Comparative Example 1]

In accordance with the above-described testing method, an attempt was made to modify gold microparticles with the PMOVE-CHO obtained in Synthesis Example 9. However, the polymer had low reactivity with gold microparticles, and thus, did not allow an end thereof to bind with gold microparticles, failing to modify the gold microparticles.

### [Comparative Example 2]

In accordance with the above-described testing method, an attempt was made to modify gold microparticles with the PNBVE-SH obtained in Synthesis Example 10. However, the polymer was hydrophobic, and thus, was condensed, failing to modify the gold microparticles.

### [Comparative Example 3]

Gold microparticles coated with PEG (PEG-coated gold microparticles) were obtained in the same manner as in Example 1, using PEG-SH (Mn = 5000, Mw/Mn = 1.02; manufactured by NOF Corporation).

### [Cytotoxicity Test]

The cell-adhesive compositions containing the PMOVE-coated gold microparticles obtained in Examples 1 to 5 and the cell-adhesive composition containing the PEG-coated gold microparticles obtained in Comparative Example 3 underwent the following cytotoxicity test.

### Testing method:

Human cervical cancer cells Hela-luc suspended in a DEME culture medium were seeded at 5,000 cells/well in a 96-well plate, and cultured for 24 hours. Then, the different kinds of polymer-coated gold microparticles obtained in Examples 1 to 5 and Comparative Example 3 were mixed at a concentration of 0.05 mg/mL. After 24 hours, 10 µL of Cell Counting Kit-8 (Dojindo Laboratories) was mixed, and the resulting mixture was left to stand for 2 hours. Then, an absorption wavelength at 450 nm was measured using an absorbance measurement device (Model No.: Spark 20M Multimode Microplate Reader, manufactured by Tecan Japan Co., Ltd.), and the cell survival rate was calculated.

As the results of the above-described test, none of the cell-adhesive compositions in Examples 1 to 5 and Comparative Example 3 was recognized to be toxic to the cell. The compositions had excellent biocompatibility.

### [Cell Adhesiveness Test]

The cell-adhesive compositions containing the PMOVE-coated gold microparticles obtained in Examples 1 to 5 and the cell-adhesive composition containing the PEG-coated gold microparticles obtained in Comparative Example 3 underwent the following cell adhesiveness test.

### Testing method:

Human cervical cancer cells Hela-luc suspended in a DEME culture medium were seeded at 50,000 cells/well in a 12-well plate, and cultured for 24 hours. Then, the different kinds of polymer-coated gold microparticles obtained in Examples 1 to 5 and Comparative Example 3 were added thereto at 0.05 mg/mL. After 24 hours, the culture medium was removed, and the resulting mixture was washed well with PBS. The cells and the gold microparticles bonded to the cells were dissolved in nitric acid, and the concentration of the gold was quantitated, using an ICP emission spectrophotometer (Model No.: iCAP PRO series ICP-OES, manufactured by Thermo Fisher Scientific Inc.).

As the results of the above-described test, the cell-adhesive compositions in Examples 1 to 5 were recognized to be bonded to the cells, and had excellent cell adhesiveness. On the other hand, the cell-adhesive composition in Comparative Example 3 was not recognized to be bonded to the cells. The composition had poor cell adhesiveness.

## Claims

1. A cell-adhesive composition comprising an oxyethylene chain-containing polymer represented by the following general formula (1): 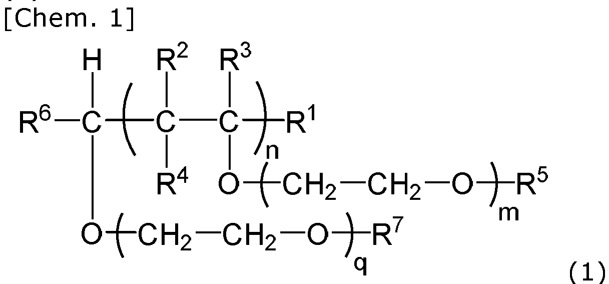
(wherein, in the general formula (1), R¹ is -(CH₂)ₚ-SH, -(CH₂)ₚ-N₃, -(CH₂)ₚ-NH₂, or -(CH₂)ₚ-COOH, and p is an integer of 1 to 5;
R² to R⁴ are each independently hydrogen or a C₁₋₁₀ alkyl group;
R⁵ is a C₁₋₁₀ alkyl group;
R⁶ is hydrogen or a C₁₋₅ alkyl group;
R⁷ is a C₁₋₁₀ alkyl group;
n is an integer of 5 to 1000;
m is an integer of 1 to 10; and
q is an integer of 0 to 5).

2. The cell-adhesive composition according to claim 1, wherein, in the general formula (1), R¹ is -(CH₂)ₚ-SH, and p is an integer of 1 to 3;
R² to R⁴ are each independently hydrogen or a C₁₋₄ alkyl group;
R⁵ is a C₁₋₄ alkyl group;
R⁶ is a C₁₋₂ alkyl group;
R⁷ is a C₁₋₇ alkyl group;
n is an integer of 10 to 500;
m is an integer of 1 to 5; and
q is an integer of 0 to 2.

3. The cell-adhesive composition according to claim 1, wherein, in the general formula (1), R¹ is -(CH₂)ₚ-N₃, and p is an integer of 1 to 3;
R² to R⁴ are each independently hydrogen or a C₁₋₄ alkyl group;
R⁵ is a C₁₋₄ alkyl group;
R⁶ is a C₁₋₂ alkyl group;
R⁷ is a C₁₋₇ alkyl group;
n is an integer of 10 to 500;
m is an integer of 1 to 5; and
q is an integer of 0 to 2.

4. The cell-adhesive composition according to claim 1, wherein, in the general formula (1), R¹ is -(CH₂)ₚ-NH₂, and p is an integer of 1 to 3;
R² to R⁴ are each independently hydrogen or a C₁₋₄ alkyl group;
R⁵ is a C₁₋₄ alkyl group;
R⁶ is a C₁₋₂ alkyl group;
R⁷ is a C₁₋₇ alkyl group;
n is an integer of 10 to 500;
m is an integer of 1 to 5; and
q is an integer of 0 to 2.

5. The cell-adhesive composition according to claim 1, wherein, in the general formula (1), R¹ is -(CH₂)ₚ-COOH, and p is an integer of 1 to 3;
R² to R⁴ are each independently hydrogen or a C₁₋₄ alkyl group;
R⁵ is a C₁₋₄ alkyl group;
R⁶ is a C₁₋₂ alkyl group;
R⁷ is a C₁₋₇ alkyl group;
n is an integer of 10 to 500;
m is an integer of 1 to 5; and
q is an integer of 0 to 2.

6. The cell-adhesive composition according to any one of claims 1 to 5, wherein the number-average molecular weight (Mn) of the oxyethylene chain-containing polymer is 500 to 100,000.

7. The cell-adhesive composition according to any one of claims 1 to 5, wherein the ratio of the weight-average molecular weight (Mw) of the oxyethylene chain-containing polymer to the number-average molecular weight (Mn) of the polymer is 1.0 or more and 3.0 or less.

8. Microparticles coated with an oxyethylene chain-containing polymer,
wherein the oxyethylene chain-containing polymer is represented by the following general formula (1): 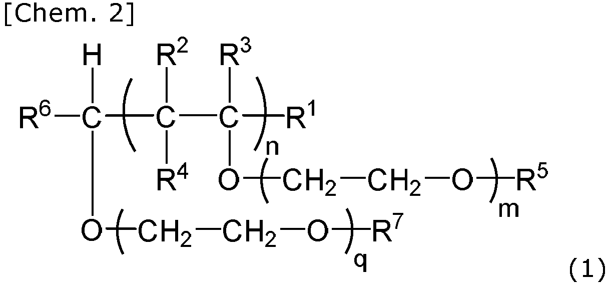
(wherein, in the general formula (1), R¹ is -(CH₂)ₚ-SH, -(CH₂)ₚ-N₃, -(CH₂)ₚ-NH₂, or -(CH₂)ₚ-COOH, and p is an integer of 1 to 5;
R² to R⁴ are each independently hydrogen or a C₁₋₁₀ alkyl group;
R⁵ is a C₁₋₁₀ alkyl group;
R⁶ is hydrogen or a C₁₋₅ alkyl group;
R⁷ is a C₁₋₁₀ alkyl group;
n is an integer of 5 to 1000;
m is an integer of 1 to 10; and
q is an integer of 0 to 5).

9. The polymer-coated microparticles according to claim 8, wherein, in the general formula (1), R¹ is -(CH₂)ₚ-SH, and p is an integer of 1 to 3;
R² to R⁴ are each independently hydrogen or a C₁₋₄ alkyl group;
R⁵ is a C₁₋₄ alkyl group;
R⁶ is a C₁₋₂ alkyl group;
R⁷ is a C₁₋₇ alkyl group;
n is an integer of 10 to 500;
m is an integer of 1 to 5; and
q is an integer of 0 to 2.

10. The polymer-coated microparticles according to claim 8, wherein, in the general formula (1), R¹ is -(CH₂)ₚ-N₃, and p is an integer of 1 to 3;
R² to R⁴ are each independently hydrogen or a C₁₋₄ alkyl group;
R⁵ is a C₁₋₄ alkyl group;
R⁶ is a C₁₋₂ alkyl group;
R⁷ is a C₁₋₇ alkyl group;
n is an integer of 10 to 500;
m is an integer of 1 to 5; and
q is an integer of 0 to 2.

11. The polymer-coated microparticles according to claim 8, wherein, in the general formula (1), R¹ is -(CH₂)ₚ-NH₂, and p is an integer of 1 to 3;
R² to R⁴ are each independently hydrogen or a C₁₋₄ alkyl group;
R⁵ is a C₁₋₄ alkyl group;
R⁶ is a C₁₋₂ alkyl group;
R⁷ is a C₁₋₇ alkyl group;
n is an integer of 10 to 500;
m is an integer of 1 to 5; and
q is an integer of 0 to 2.

12. The polymer-coated microparticles according to claim 8, wherein, in the general formula (1), R¹ is -(CH₂)ₚ-COOH, and p is an integer of 1 to 3;
R² to R⁴ are each independently hydrogen or a C₁₋₄ alkyl group;
R⁵ is a C₁₋₄ alkyl group;
R⁶ is a C₁₋₂ alkyl group;
R⁷ is a C₁₋₇ alkyl group;
n is an integer of 10 to 500;
m is an integer of 1 to 5; and
q is an integer of 0 to 2.

13. The polymer-coated microparticles according to any one of claims 8 to 12, wherein the number-average molecular weight (Mn) of the oxyethylene chain-containing polymer is 500 or more and 100,000 or less.

14. The polymer-coated microparticles according to any one of claims 8 to 12, wherein the ratio of the weight-average molecular weight (Mw) of the oxyethylene chain-containing polymer to the number-average molecular weight (Mn) of the polymer is 1.0 or more and 3.0 or less.

15. The polymer-coated microparticles according to any one of claims 8 to 12, wherein the microparticles are inorganic microparticles of at least one selected from the group consisting of gold, platinum, silver, and silicon dioxide.

16. The polymer-coated microparticles according to any one of claims 8 to 12, wherein the average particle diameter of the microparticles is 10 nm or more and 500 nm or less.

17. A cell-adhesive composition comprising polymer-coated microparticles according to any one of claims 8 to 12.
